# EUROPÄISCHE PATENTANMELDUNG

(11) **EP 4 537 740 A1**
(43) Veröffentlichungstag der Anmeldung: **16.04.2025**
(21) Anmeldenummer: 23203017.1
(22) Anmeldetag: 11.10.2023
(51) Int. Cl.: A61B 5/00

(54) **DIGITALES DERMATOSKOP**

(71) Anmelder: Magnosco GmbH, 12489 Berlin (DE)
(72) Erfinder: Fett, Axel, 12047 Berlin (DE); Seifert, Christoph, 15732 Eichwalde (DE); Stankovic, Goran, 12051 Berlin (DE); Patrovsky, Fabian, 12623 Berlin (DE)
(74) Vertreter: Gulde & Partner

(57) **Zusammenfassung**

Digitales Dermatoskop (10) zur Hautuntersuchung, wobei das digitale Dermatoskop (10) als Handgerät ausgebildet ist, das digitale Dermatoskop (10) umfassend ein mit der Hand umgreifbares Griffteil (12) und ein an das Griffteil (12) anschließen-des, eine Bildgebungseinheit (20) aufweisendes Kopfteil (14), wobei die Bildgebungseinheit (20) ein an einer Unterseite des Kopfteils (14) angeordnetes Bildgebungseinheitsgehäuse (22), einen in dem Bildgebungseinheitsgehäuse (22) aufgenommenen Bildsensor (24), sowie ein Objektiv (26) aufweist, wobei endseitig an dem Bildgebungseinheitsgehäuse (22) ein auswechselbarer Abstandsvorsatz (30) angeordnet ist, der bei einer Hautuntersuchung die zu untersuchende Haut kontaktiert, das digitale Dermatoskop (10) weiter umfassend eine Steuereinheit (50) sowie ein an einer Oberseite des Kopfteils (14) angeordnetes erstes Display (40), wobei die Steuereinheit (50) dazu ausgebildet ist, das erste Display (40) zur Darstellung von mittels der Bildgebungseinheit (20) aufgenommenem Bildern eines zu untersuchenden Hautabschnitts anzusteuern.

## Beschreibung

Die Erfindung betrifft ein digitales Dermatoskop zur Untersuchung der Haut eines Patienten.

Die Dermatoskopie, auch Dermoskopie oder Auflichtmikroskopie genannt, ermöglicht Ärzten die schnelle Darstellung von Hautstrukturen bis zur papillären Dermis. Die bei der Dermatoskopie sichtbaren Hautmuster sind meist auf Pigment- und Gefäßstrukturen zurückzuführen.

Die Dermatoskopie ermöglicht im Vergleich zur Untersuchung mit bloßem Auge eine bessere Unterscheidung des (frühen) malignen Melanoms (sogenannter schwarzer Hautkrebs) von seinen gutartigen Simulatoren. Je nach Lokalisation und Krankheitszustand werden der Dermatoskopie unterschiedliche Namen gegeben: Die Trichoskopie wird bei Kopfhaut- und Haarerkrankungen, die Inflammoskopie bei entzündlichen Erkrankungen und die Entomodermoskopie zur Untersuchung von Befall und Infektionszuständen mittels Dermatoskopie eingesetzt. So kann der Zustand der Haut und ihrer Veränderungen betrachtet und eine Früherkennung von bösartigen Tumoren der Haut, wie beispielsweise dem malignen Melanom erreicht werden.

Es werden bislang zumeist analoge Dermatoskope verwendet. Diese analogen Dermatoskope sind in der Regel handgehaltene Lupen mit entsprechender Vergrößerung und eingebautem Licht zur Ausleuchtung des Untersuchungsfeldes. Zum Zwecke der Hautuntersuchung wird ein solches analoges Dermatoskop mit einer ersten Seite seines Kopfteils auf den zu untersuchenden Hautabschnitt aufgesetzt, es wird Licht mittels des Dermatoskops auf den zu untersuchenden Hautabschnitt gestrahlt und von der Haut reflektiert. Das reflektierte Licht verlässt das analoge Dermatoskop, nach Durchgang durch eine Optik, auf der gegenüberliegenden Seite des Kopfteils und gelangt somit zu dem Auge einer die Untersuchung durchführenden Person.

Analoge Dermatoskope weisen eine Reihe von Nachteilen auf. So können keine Aufnahmen der Haut als Bilder gespeichert und damit weder dem Patienten gezeigt noch für eine langfristige Dokumentation verwendet werden. Die die Untersuchung durchführende Person kann lediglich die Einzelheiten des untersuchten Hautabschnitts in Worten beschreiben und diese als Dokumentation festhalten. Insbesondere eine Langzeitverfolgbarkeit ist dadurch schwer möglich und relativ ungenau. Auch kann die Haut mittels eines analogen Dermatoskops nur bedingt vergrößert werden, insbesondere nur optisch und üblicherweise nur mit einem fixen Vergrößerungsfaktor.

Grundsätzlich besteht die Möglichkeit, ein analoges Dermatoskop gemeinsam mit einer digitalen Kamera, beispielsweise einem Smartphone zu verwenden. So könnte das Smartphone beispielsweise mit seinem Kameraobjektiv auf das Kopfteil des analogen Dermatoskops aufgesetzt werden, sodass das von der Haut reflektierte Licht nicht in das Auge der die Untersuchung durchführenden Person, sondern in das Kameraobjektiv einfällt. Dies erfordert jedoch die Verwendung eines Zweitgeräts, was umständlich ist. Zudem sind solche digitalen Kameras nicht für die Hautuntersuchung ausgelegt und insbesondere auch nicht für die Zusammenarbeit mit der Optik des analogen Dermatoskops. Es kann daher zu Problemen bei dem Scharfstellen der Optik und/oder zu Abbildungsfehlern kommen. Zudem ist die Verwendung insbesondere eines Smartphones ohne spezielle technische Lösungen auch datenschutzrechtlich problematisch, da somit möglicherweise unbefugte Dritte Zugang zu sensiblen Patientendaten erhalten.

Ausgehend von dem erläuterten Stand der Technik stellt sich die Aufgabe, ein Dermatoskop bereitzustellen, welches die genannten Nachteile nicht aufweist, welches insbesondere eine einfachere Untersuchung der Haut und/oder Hautanhangsgebilde sowie eine zuverlässigere Diagnose ermöglicht.

Die Erfindung löst die Aufgabe durch ein Dermatoskop gemäß Anspruch 1. Vorteilhafte Ausgestaltungen sind Gegenstand der abhängigen Ansprüche, der Beschreibung sowie der Figuren.

Die Erfindung betrifft ein digitales Dermatoskop zur Hautuntersuchung, wobei das digitale Dermatoskop als Handgerät ausgebildet ist, das digitale Dermatoskop umfassend ein mit der Hand umgreifbares Griffteil und ein an das Griffteil anschließendes, eine Bildgebungseinheit aufweisendes Kopfteil, wobei die Bildgebungseinheit ein an einer Unterseite des Kopfteils angeordnetes Bildgebungseinheitsgehäuse, einen in dem Bildgebungseinheitsgehäuse aufgenommenen Bildsensor, sowie ein Objektiv aufweist, wobei endseitig an dem Bildgebungseinheitsgehäuse ein auswechselbarer Abstandsvorsatz angeordnet ist, der bei einer Hautuntersuchung die zu untersuchende Haut kontaktiert, wobei das digitale Dermatoskop weiter umfasst: eine Steuereinheit sowie ein an einer Oberseite des Kopfteils angeordnetes erstes Display, wobei die Steuereinheit dazu ausgebildet ist, das erste Display zur Darstellung von mittels der Bildgebungseinheit aufgenommenem Bildern eines zu untersuchenden Hautabschnitts anzusteuern.

Das erfindungsgemäße Dermatoskop ist digital, da es dazu ausgebildet ist, digitale Bilder der Haut und/oder Hautanhangsgebilde aufzunehmen und diese Bilder auf dem ersten Display anzuzeigen sowie bevorzugt die Bilder digital zu speichern. Im Weiteren wird auf die Hautuntersuchung mittels des erfindungsgemäßen Dermatoskops eingegangen, wobei stets auch die Untersuchung der Nägel oder Haare umfasst ist. Das digitale Dermatoskop weist, ähnlich dem Aufbau eines analogen Dermatoskops, einen Griffteil auf sowie einen Kopfteil auf. Das Griffteil kann von einer Bedienperson gegriffen werden, um mit dem digitalen Dermatoskop eine Hautuntersuchung durchzuführen. Zur Hautuntersuchung wird das digitale Dermatoskop mit seinem Abstandsvorsatz auf den zu untersuchenden Hautabschnitt eines Patienten aufgesetzt, sodass der Abstandsvorsatz mit seinem freien, von dem Kopfteil wegweisenden Ende die Hautoberfläche kontaktiert. Der Abstandsvorsatz weist an dem freien Ende eine Öffnung auf, durch welche von dem digitalen Dermatoskop ausgesandtes Licht zu der Haut gelangen kann. Hierfür kann das digitale Dermatoskop eine bevorzugt ringförmig angeordnete LEDs (LED = Licht emittierende Diode) umfassende Beleuchtungseinheit aufweisen. Die Beleuchtungseinheit kann insbesondere eine Dunkelfeld-Beleuchtungseinheit sein. Das von dem digitalen Dermatoskop emittierte Licht wird von dem beleuchteten Hautabschnitt reflektiert, von dem Objektiv der Bildgebungseinheit aufgenommen und auf den Bildsensor fokussiert. Der Bildsensor erzeugt aus dem reflektierten Licht ein digitales Bild, welches das digitale Dermatoskop dann auf dem ersten Display anzeigt. Die Steuereinheit ist zur entsprechenden Ansteuerung der hierfür benötigten Komponenten des digitalen Dermatoskops ausgebildet. In anderen Worten ist die Steuereinheit dazu ausgebildet, dass von dem Bildsensor aufgenommene Bild auf dem ersten Display anzuzeigen und ggf. in einer Speichereinheit des digitalen Dermatoskops zu speichern. Das somit auf dem ersten Display angezeigte Bild des zu untersuchenden Hautabschnitts kann von einer die Untersuchung durchführenden Person (im Folgenden auch Bedienperson genannt) betrachtet werden. Die Steuereinheit kann einen Prozessor umfassen. Die Steuereinheit kann als "system-on-module" (SOM) oder "system-on-chip" (SOC) ausgebildet sein, also als integrierter Schaltkreis. Insbesondere kann das SOM/SOC in Kombination mit einem Carrierboard verwendet werden, worüber einzelne Funktionen und/oder Schnittstellen des digitalen Dermatoskops realisiert werden können. Beispielsweise kann das SOM/SOC über vordefinierte Pins mit dem Carrierboard verbunden werden. Mit einem solchen Aufbau kann der benötigte Bauraum reduziert und zudem eine Austauschbarkeit des SOM/SOC gegeben sein, beispielsweise um später ein leistungsfähigeres SOM/SOC nachzurüsten.

Die Bildgebungseinheit ist in oder an dem Kopfteil des digitalen Dermatoskops angeordnet. Insbesondere ist das Bildgebungseinheitsgehäuse und damit die Bildgebungseinheit an einer Unterseite des Kopfteils angeordnet bzw. erstreckt sich von dem Kopfteil nach unten fort, wobei die Unterseite hierbei die im Verwendungszustand zur Haut zeigende Seite des Kopfteils meint. Die Bildgebungseinheit erstreckt sich also zur Haut hin. Die Bildgebungseinheit ist insbesondere, bevorzugt über das Bildgebungseinheitsgehäuse, in das Kopfteil integriert. Die Bildgebungseinheit kann beispielsweise mit dem Kopfteil, bevorzugt über das Bildgebungseinheitsgehäuse, verschraubt oder verschweißt sein. Der Bildsensor kann beispielsweise ein 8 MP bis 12 MP-Sensor sein, bevorzugt ein 12 MP-Sensor sein, also eine Auflösung von 12 Megapixeln aufweisen, bevorzugt von 12 Megapixeln. Damit kann die Haut ausreichend hoch aufgelöst werden, um eventuelle Hauterkrankungen oder Dermatosen gut erkennen zu können. Natürlich kann auch eine höhere Auflösung möglich sein. Das Objektiv weist bevorzugt eine Festbrennweite auf, beispielsweise eine Festbrennweite von 8 mm. Das Objektiv kann eine Blende von beispielsweise f/4 aufweisen. Das Bildgebungseinheitsgehäuse und damit die Bildgebungseinheit ist mit einem ersten Ende mit dem Kopfteil des digitalen Dermatoskops verbunden und mit einem dem ersten Ende gegenüberliegenden zweiten Ende mit dem Abstandsvorsatz.

Der Abstandsvorsatz stellt über seine Längsausdehnung entlang der optischen Achse für die Bildaufnahme einen vordefinierten Abstand der Bildgebungseinheit, insbesondere des Objektivs, zur Haut sicher. Dies ist insbesondere notwendig, wenn die Bildgebungseinheit des Dermatoskops aufgrund einer eventuellen Festbrennweite des Objektivs nur in dem durch den Abstandsvorsatz erreichten, fixen Abstand zur untersuchenden Haut scharfstellen kann. Das digitale Dermatoskop, bzw. das Objektiv desselben, kann also insbesondere eine Festbrennweite aufweisen. Festbrennweiten haben insbesondere den Vorteil eines besseren Auflösungsvermögens. Mittels des Abstandsvorsatzes wird somit sichergestellt, dass der zu untersuchende Hautabschnitt in der Fokusebene der Bildgebungseinheit angeordnet ist. Hierfür muss das digitale Dermatoskop mit dem freien Ende des Abstandsaufsatzes auf die Haut aufgesetzt werden, also die Haut kontaktieren. Der Abstandsvorsatz kann über den Durchmesser der insbesondere kreisförmigen Öffnung an seinem freien Ende einen Aufnahmedurchmesser (oder in anderen Worten einen Bildbetrachtungsdurchmesser) für das aufzunehmende Bild vorgeben.

Der Abstandsvorsatz ist auswechselbar, kann insbesondere durch einen Abstandsvorsatz mit anderen bildbeeinflussenden Eigenschaften, wie einem Abstandsvorsatz mit einem anderen Öffnungsdurchmesser, ersetzt werden. Über unterschiedliche Abstandsvorsätze können somit Hautabschnitte unterschiedlicher Größe untersucht werden. Beispielsweise können relativ großflächige Untersuchungen mittels eines Abstandsvorsatzes mit einem großen Öffnungsdurchmesser und relativ kleinflächige Untersuchungen, wie die Hautuntersuchung von engen Körperstellen, mittels kleinerer Abstandsvorsätze durchgeführt werden. Der Abstandsvorsatz ist an das freie Ende des Bildgebungseinheitsgehäuse bevorzugt aufgesetzt, alternativ eingesetzt. Das Bildgebungseinheitsgehäuse kann hierfür an seinem freien Ende eine insbesondere kreisförmige Aussparung aufweisen und der Abstandsvorsatz ein korrespondierend zu der kreisförmigen Aussparung geformtes Ansatzstück, sodass, wenn der Abstandsvorsatz auf das Bildgebungseinheitsgehäuse aufgesetzt wird, das Ansatzstück mit der kreisförmigen Aussparung in Überlappung kommt. Die Aussparung kann jedoch auch eine andere Form aufweisen, beispielsweise eine rechteckige, ovale, oder sternförmige Form. Der Abstandsvorsatz hält bevorzugt magnetisch an dem Bildgebungseinheitsgehäuse.

Das erste Display ist ein elektronisches Display, insbesondere ein LCD-Display, beispielsweise mit LED-Hintergrundlicht. Das erste Display ist in dem Kopfteil des Dermatoskops derart angeordnet, dass es von der Oberseite des Kopfteils und damit des digitalen Dermatoskops abgelesen werden kann. Die Oberseite bezeichnet dabei die der Bedienperson während der Untersuchung zugewandte Seite des digitalen Dermatoskops. Das erste Display kann von einem Deckglas abgedeckt sein, es kann also ein Deckglas oberhalb des ersten Displays angeordnet sein. Das erste Display ist bevorzugt in einer optischen Achse der Bildgebungseinheit (insbesondere des Objektivs) angeordnet, insbesondere derart, dass die optische Achse durch die Mitte des ersten Displays verläuft. Dies macht die Verwendung des digitalen Dermatoskops besonders intuitiv, da die Bedienperson, wie bei den ihr bereits bekannten analogen Dermatoskopen, somit den Eindruck erhält in gerader Linie "durch das Dermatoskop hindurch zu blicken". Weiter bevorzugt weist das erste Display eine (kreis-)runde Form auf, was aufgrund der Ähnlichkeit zu einem analogen Dermatoskop ebenfalls zu einer intuitiven Bedienung beiträgt. Besonders Hauterkrankungen oder Dermatosen, oder maligne Melanome die eine, können so effektiv untersucht werden. Das erste Display kann als nur bildgebend ausgebildet sein, also nicht als Bedienelement. Das erste Display ist jedoch bevorzugt ein Touch-Display, also über einen oder mehrere Finger der Bedienperson bedienbar. So kann das erste Display dazu ausgebildet sein, von der Bedienperson mittels ihrer Finger auf der Displayoberfläche durchgeführte Touch-Gesten zu erkennen und in Reaktion auf das Erkennen einer vordefinierten Touch-Geste eine Aktion durchzuführen. Bevorzugt ist das Display demnach dazu ausgebildet eine Touch-Geste zu erkennen, die eine Zoom-Funktion auslöst. Somit kann die Bedienperson unmittelbar auf dem ersten Display ein dort dargestelltes Bild des zu untersuchenden Hautabschnitts mittels Touch-Geste vergrößern oder verkleinern, insbesondere unter Anwendung eines Digital-Zooms. Beispielsweise kann hierfür eine "pinch-to-zoom"-Geste vorgesehen sein, bei welcher durch das Aufeinanderzubewegen zweier Finger hinausgezoomt und durch das Voneinanderfortbewegen zweier Finger hineingezoomt wird. Das erste Display nimmt den "Lupencharakter" eines analogen Dermatoskops auf und überträgt diesen für die Bedienperson in die digitale Welt.

Das erfindungsgemäße digitale Dermatoskop ist, wie erwähnt, dazu ausgebildet, digitale Bilder der Haut aufzunehmen und diese Bilder auf dem ersten Display anzuzeigen. Das digitale Dermatoskop kann insbesondere mehrere Bilder pro Sekunde aufnehmen und diese auf dem ersten Display als Live-Ansicht, also als Live-Video, anzeigen. Auch können einzelne Standbilder der Haut aufgenommen und auf dem ersten Display angezeigt werden. Bevorzugt ist das digitale Dermatoskop dazu ausgebildet, Aufnahmen der Haut als digitale Bilder zu speichern, beispielsweise in einer Speichereinheit des digitalen Dermatoskops zu hinterlegen. Weiter bevorzugt ist das digitale Dermatoskop, insbesondere die Steuereinheit dazu ausgebildet, das aufgenommene Bild auf dem ersten Display in Falschfarben oder in schwarz-weiß anzuzeigen. Weiter bevorzugt ist das digitale Dermatoskop dazu ausgebildet, einen Kontrastwert des aufgenommenen und auf dem ersten Display angezeigten Bildes zu verändern.

Die aufgenommenen Bilder können auf dem ersten Display nicht nur während der Untersuchung angezeigt werden, sondern auch wenn das digitale Dermatoskop von der Haut weggenommen wird. Insbesondere kann das digitale Dermatoskop dazu ausgebildet sein, auch Bilder anzuzeigen, welche bei früheren Untersuchungen aufgenommen worden sind. Es können somit dem Patienten die untersuchten Hautabschnitte unmittelbar über das erste Display gezeigt werden, ohne die Notwendigkeit das Dermatoskop auf der Haut aufgesetzt zu lassen. Zudem ist eine langfristige Dokumentation des untersuchten Hautabschnitts möglich, zum Beispiel durch Speichern der Bilder mehrerer Untersuchungen in dem digitalen Dermatoskop selbst oder durch Übertragen der Bilder an ein anderweitiges elektronisches Gerät, beispielsweise an einen Computer, an ein Tablet oder ein Smartphone. Die Bilder können mit einem anderen Arzt geteilt oder auch maschinell, beispielsweise mittels künstlicher Intelligenz, zum Zwecke der Diagnose ausgewertet werden. Zudem können einzelne Bildausschnitte eines mittels des digitalen Dermatoskops aufgenommenen Bildes, abhängig von der Auflösung des Bildes, während der Untersuchung, beispielsweise mittels eines Digital-Zooms, oder auch im Nachgang zur Untersuchung vergrößert werden. Das digitale Dermatoskop ist damit nicht festgelegt auf einen bauteilbedingten fixen Vergrößerungsfaktor. Das digitale Dermatoskop ist besonders kompakt und einfach aufgebaut, da die Bildgebungseinheit im Kopfteil des digitalen Dermatoskops angeordnet, also in das digitale Dermatoskop integriert ist. Zudem kann bei entsprechender Ausbildung der Steuereinheit das aufgenommene Bild auf dem ersten Display in abgewandelter Form angezeigt werden, beispielsweise in Falschfarben oder mit verändertem Kontrast, was einer Bedienperson das Erkennen von Hauterkrankungen oder Dermatosen erleichtern kann. Das erfindungsgemäße digitale Dermatoskop ermöglicht eine einfachere, benutzerfreundlichere Untersuchung der Haut sowie eine zuverlässigere Diagnose.

Nach einer Ausgestaltung weist das erste Display eine zu der Form des Kopfteils korrespondierende Form auf. Nach einer diesbezüglichen Ausgestaltung weisen das Kopfteil und/oder das erste Display eine (kreis)runde Form auf. Insbesondere weisen das erste Display und das Kopfteil zueinander korrespondierende (kreis)runde Formen auf. Wie erwähnt, trägt ein rundes erstes Display aufgrund der Ähnlichkeit zu einem analogen Dermatoskop zu einer intuitiven Bedienung bei und ermöglicht zudem eine besonders effektive Hautuntersuchung. Insbesondere ermöglicht dies eine besonders effektive Untersuchung von Hauterkrankungen oder Dermatosen, wie beispielsweise von malignen Melanomen, die eine zumindest annähernd runde Form aufweisen. Ein rundes Display nimmt insbesondere den "Lupencharakter" auf, übersetzt also das analoge Dermatoskop für den Anwender in die digitale Welt. Eine weitere Ähnlichkeit zu einem analogen Dermatoskop wird über das in korrespondierender Weise (rund) geformte Kopfteil erreicht, da dieses bei analogen Dermatoskopen ebenfalls rund ist. Das erste Display nimmt bevorzugt einen Großteil der Oberfläche des Kopfteils ein, insbesondere wenigstens 80%, bevorzugt mindestens 90% der Oberfläche des Kopfteils. Dies ermöglicht eine große Displayfläche und damit eine großflächige Darstellung des zu untersuchenden Hautabschnitts bei relativ kompakter Größe des Kopfteils. Zudem ist dies ästhetisch ansprechend.

Nach einer Ausgestaltung ist die Steuereinheit dazu ausgebildet, eine Live-Ansicht des zu untersuchenden Hautabschnitts darzustellen. Das digitale Dermatoskop bzw. dessen Bildgebungseinheit kann also, wie erwähnt, bevorzugt mehrere Bilder pro Sekunde aufnehmen und diese auf dem ersten Display als Live-Ansicht, also als Live-Video, anzeigen. Das digitale Dermatoskop kann beispielsweise ausgebildet sein, mindestens 15 Bilder pro Sekunde, bevorzugt 24 Bilder pro Sekunde, weiter bevorzugt mindestens 30 Bilder pro Sekunde, weiter bevorzugt mindestens 60 Bilder pro Sekunde aufzunehmen. Somit kann das digitale Dermatoskop unmittelbar, also ohne Zeitverzögerung, die zu untersuchende, momentan unter dem Abstandsvorsatz befindliche Haut anzeigen. Somit können zu untersuchende Hauterkrankungen oder Dermatosen der Haut schnell und einfach aufgefunden werden. Auch hiermit wird insoweit ein analoges Dermatoskop simuliert. Allerdings ermöglicht das digitale Dermatoskop beispielsweise das Speichern der Bilder oder Videos sowie auch die längerfristige Anzeige eines Standbilds der Hauterkrankungen oder Dermatosen, auch wenn das digitale Dermatoskop nicht länger auf der Haut aufliegt.

Nach einer Ausgestaltung ist die Steuereinheit dazu ausgebildet, ein auf dem ersten Display dargestelltes Bild des zu untersuchenden Hautabschnitts in Reaktion auf eine Bedieneingabe mittels eines digitalen Zooms zu vergrößern. Das digitale Dermatoskop kann dazu ausgebildet sein, eine entsprechende Bedieneingabe zu empfangen. Beispielsweise kann das digitale Dermatoskop ein Bedienelement umfassen zum Empfang einer Bedieneingabe zum Auslösen einer Zoom-Funktion. Ein solches Bedienelement kann durch ein weiteres Display gegeben sein, insbesondere durch das später noch erläuterte, im Griffteil angeordnete zweite Display. Bevorzugt ist jedoch das erste Display als Touch-Display ausgebildet und zum Empfang einer solchen Bedieneingabe ausgebildet, wie bereits erwähnt, Insbesondere kann das digitale Dermatoskop dazu ausgebildet sein, eine solche Bedieneingabe während der Live-Ansicht der Haut auf dem ersten Display zu empfangen, sodass "live" in das Bild hinein- oder hinausgezoomt werden kann. Eine solche Art der Bedienung ist besonders intuitiv. Der digitale Zoom ermöglicht eine Vergrößerung (oder Verkleinerung) des zu untersuchenden Hautabschnitts währen der Untersuchung. Hierdurch können Details für die Bedienperson sichtbar werden, welche mit einem herkömmlichen analogen Dermatoskop möglicherweise nicht erkennbar wären. Dennoch kann eine vorteilhafte Festbrennweite erhalten bleiben.

Nach einer Ausgestaltung ist das erste Display dazu ausgebildet, das durch die Bildgebungseinheit aufgenommene Bild in anderen Farben, insbesondere in Falschfarben oder in schwarz-weiß darzustellen. Die erfindungsgemäße Digitalisierung des Dermatoskops ermöglicht eine Darstellung der Haut nicht nur in Echtfarben, sondern auch verfälscht oder in schwarz-weiß, was einige Hauteigenheiten besonders hervorheben kann. Es können somit eventuelle Hauterkrankungen oder Dermatosen der Haut besser erkannt werden.

Nach einer Ausgestaltung umfasst das digitale Dermatoskop ein an einer Oberseite des Griffteils angeordnetes als Touch-Display ausgebildetes zweites Display zur Darstellung von Bedienelementen zum Bedienen des digitalen Dermatoskops. Das zweite Display ist ebenfalls ein elektronisches Display. Das zweite Display ist dazu ausgebildet, ein oder mehrere Bedienelemente darzustellen, beispielsweise ein Bedienelement zum Ausführen der erwähnten Zoom-Funktion. Bevorzugt ist das zweite Display dazu ausgebildet, eines oder mehrere der folgenden Bedienelemente darzustellen: Ein Bedienelement zur Darstellung des durch die Bildgebungseinheit aufgenommenen Bildes auf dem ersten Display in anderen Farben, insbesondere in Falschfarben oder schwarz-weiß, ein Bedienelement zur Änderung des Kontrastes des auf dem ersten Display dargestellten Bildes, ein Bedienelement zum Anzeigen von zuvor aufgenommenen Bildern. Auch kann das zweite Display zur Anzeige von Statusinformationen des digitalen Dermatoskops ausgebildet sein, beispielsweise zur Darstellung eines aktuellen Batteriezustands/Ladezustands einer Batterie des digitalen Dermatoskops, zur Darstellung einer aktuellen Uhrzeit und/oder zu Darstellung einer Empfangsqualität eines WLAN-Signals (WLAN = Wireless Local Area Network). Über ein solches zweites Display kann die Vielzahl von neuartigen Funktionen des digitalen Dermatoskops in einfacher und intuitiver Weise bedient werden. Zum Schutz des zweiten Displays kann oberhalb des zweiten Displays ein Deckglas angeordnet sein. Dies kann insbesondere dasselbe Deckglas sein, welches auch für das erste Display vorgesehen ist.

Nach einer Ausgestaltung weist das zweite Display eine zu der Form des Griffteils korrespondierende Form auf. Insbesondere weist das Griffteil und/oder das zweite Display eine rechteckige Form auf. Insbesondere weisen das zweite Display und das Griffteil zueinander korrespondierende rechteckige Formen auf. Eine rechteckige Form des Griffteils meint hierbei insbesondere eine plane, rechteckige Oberfläche des Griffteils. Eine rechteckige Form des Griffteil ist zwar in Abweichung von den üblicherweise zylindrischen Griffteilen bekannter analoger Dermatoskope, erlaubt aber die effiziente Bedienung des digitalen Dermatoskops über das zweite Display. Das zweite Display kann sich beispielsweise über mindestens 40% der Oberseite des Griffteils erstrecken, bevorzugt über mindestens 50%, besonders bevorzugt über mindestens 60%. Dies ermöglicht die gleichzeitige Darstellung mehrerer Bedienelemente und lässt gleichzeitig genügend Platz zum Umgreifen des Griffteils mit der Hand ohne versehentliches Bedienen eines der Bedienelemente.

Nach einer Ausgestaltung weist das digitale Dermatoskop einen das Griffteil und das Kopfteil einfassenden Gehäuserahmen auf. Nach einer Ausgestaltung besteht der Gehäuserahmen aus Aluminium, insbesondere aus gefrästem Aluminium. Nach einer Ausgestaltung besteht das Bildgebungseinheitsgehäuse aus Aluminium, insbesondere aus gefrästem Aluminium. Bevorzugt ist der Gehäuserahmen und/oder das Bildgebungseinheitsgehäuse einstückig aus Aluminium gefräst. Weiter bevorzugt weist der aus Aluminium bestehende Gehäuserahmen und/oder das aus Aluminium bestehende Bildgebungseinheitsgehäuse eine glasperlbestrahlte und/oder eine anodisierte Oberfläche auf. Weiter bevorzugt weist das aus Aluminium bestehende Bildgebungseinheitsgehäuse eine glasperlbestrahlte und/oder eine anodisierte Oberfläche auf. Nach einer weiteren Ausgestaltung ist der Gehäuserahmen als Kühlkörper für die darin befindlichen Elektronikkomponenten ausgebildet.

Einen solchen Kühlkörper bildet der Gehäuserahmen insbesondere dann, wenn er aus Aluminium besteht, da das Aluminium eine relativ hohe Wärmeleitfähigkeit aufweist. Ein derartiger Gehäuserahmen bzw. ein derartiges Bildgebungseinheitsgehäuse sind besonders stabil bei gleichzeitig geringem Materialbedarf. Zudem ist Aluminium ästhetisch besonders ansprechend sowie widerstandsfähig gegenüber Umwelteinflüssen, insbesondere wenn einstückig geformt und/oder glasperlbestrahlt/anodisiert. Der Gehäuserahmen und/oder das Bildgebungseinheitsgehäuse kann jedoch auch aus Magnesium, Edelstahl, oder Kunststoff bestehen.

Nach einer Ausgestaltung weist die Bildgebungseinheit mindestens einen Polarisationsfilter auf. Insbesondere kann die Bildgebungseinheit zwei Polarisationsfilter aufweisen, die bevorzugt zueinander um 90° verdreht angeordnet sind. Ein erster Polarisationsfilter kann für die Beleuchtung und ein zweiter Polarisationsfilter für die Aufnahme vorgesehen sein. Somit ist eine kreuzpolarisierte Detektion möglich. Bevorzugt weist die Bildgebungseinheit Beleuchtungselemente auf, welche ausgebildet sind, sowohl polarisiertes Licht als auch nicht polarisiertes Licht auszusenden. Insbesondere ist das digitale Dermatoskop somit zur Aufnahme eines ersten Bildes mit polarisiertem Licht sowie zur Aufnahme eines zweiten Bildes mit nicht polarisiertem Licht ausgebildet. Der Vergleich des zweiten mit dem ersten Bildes erlaubt Rückschlüsse auf mögliche Hauterkrankungen oder Dermatosen (Differenzialdiagnose). Dies ist bei dem erfindungsgemäßen digitalen Dermatoskop von besonderem Vorteil, da hier die beiden Bilder aufgezeichnet und auch im Nachgang noch untersucht werden können.

Nach einer Ausgestaltung umfasst das digitale Dermatoskop eine an einer Oberseite des digitalen Dermatoskops angeordnete, sich über das Kopfteil und das Griffteil erstreckende Deckglasscheibe. Wie bereits erwähnt, kann oberhalb des ersten Displays und/oder des zweiten Displays eine Deckglasscheibe zum Schutz der Displays angeordnet sein. Nach dieser Ausgestaltung erstreckt sich die Deckglasscheibe über die Oberseite von Kopfteil und Griffteil, bevorzugt über die im Wesentlichen gesamte Oberfläche. Dies bietet einen guten Schutz und ist ästhetisch.

Nach einer Ausgestaltung umfasst das digitale Dermatoskop eine seitlich am Griffteil angeordnete Bedientaste zum Auslösen einer vordefinierten Bedienfunktion des digitalen Dermatoskops. Die Bedientaste ist insbesondere derart angeordnet, dass sie bei Umgreifen des Griffteils mit der Hand durch den Daumen der Hand ohne Ändern der Griffposition bedient werden kann. Die Bedienfunktion kann beispielsweise ein Anschalten und/oder ein Ausschalten des digitalen Dermatoskops sein.

Nach einer Ausgestaltung umfasst das digitale Dermatoskop eine in dem Griffteil angeordnete, wiederaufladbare Batterie zur Energieversorgung der elektronischen Bauteile des digitalen Dermatoskops. Die Batterie ist bevorzugt eine Lithium-Ionen-Batterie, besonders bevorzugt eine Lithium-Polymer-Batterie. Insbesondere eine Lithium-Polymer-Batterie erlaubt aufgrund ihres festen Polymerelektrolyten ein von Platz und Gewicht, da diese sich, beispielsweise als "Pouch-Bag" besser in das Griffteil des Gehäuses fügt. Die Batterie ist bevorzugt zur Energieversorgung der Steuereinheit und des ersten Displays eingerichtet, insbesondere auch zur Versorgung des zweiten Displays, einer Speichereinheit sowie der Beleuchtungseinheit. Die Batterie kann über einen Ladeanschluss des digitalen Dermatoskops aufladbar sein. Der Ladeanschluss kann beispielsweise einen Ladeanschluss für einen Ladestecker und/oder einen Ladeanschluss zum kabellosen Laden umfassen.

Nach einer Ausgestaltung umfasst das digitale Dermatoskop eine nicht-flüchtige Speichereinheit zur elektronischen Speicherung von durch die Bildgebungseinheit aufgenommenen Bildern und/oder Videos.

Nach einer Ausgestaltung umfasst das digitale Dermatoskop einen Datenübertragungsanschluss, insbesondere einen USB-Anschluss, bevorzugt angeordnet an einem Ende des Griffteils, zum Herstellen einer Datenverbindung zwischen dem digitalen Dermatoskop und einem elektronischen Gerät, insbesondere einem Tablet, einem Computer und/oder einem Smartphone. Der Datenübertragungsanschluss kann auch zum Aufladen einer Batterie des digitalen Dermatoskops dienen, kann also auch den bereits erwähnten Ladeanschluss bilden.

Nach einer Ausgestaltung umfasst das digitale Dermatoskop eine Kommunikationseinheit zur kabellosen Kommunikation mit einem elektronischen Gerät, insbesondere einem Tablet, einem Computer und/oder einem Smartphone. Die Kommunikationseinheit kann zur Kommunikation mittels WLAN und/oder Bluetooth ausgebildet sein.

Nach einer Ausgestaltung umfasst die Bildgebungseinheit eine Beleuchtungseinheit mit mehreren ringförmig angeordneten Beleuchtungselementen zur Beleuchtung des zu untersuchenden Hautabschnitts, wie bereits angesprochen. Die Beleuchtungselemente sind bevorzugt LEDs. Nach einer Ausgestaltung sind die Beleuchtungselemente in einem Winkel zur optischen Achse angeordnet. Nach einer Ausgestaltung sind die Beleuchtungselemente winkelverstellbar zur optischen Achse angeordnet. Nach einer Ausgestaltung umfassen die Beleuchtungselemente eine erste Anzahl LEDs, beispielsweise sechs LEDs, zum Aussenden von polarisiertem Licht, bzw. zur Erzeugung von polarisiertem Licht auf dem zu untersuchenden Hautabschnitt, und eine zweite Anzahl LEDs, beispielsweise sechs LEDs, zum Aussenden von nicht polarisiertem Licht. Die erste und die zweite Anzahl sind bevorzugt identisch. Die LEDs können gleichmäßig über den Ringumfang angeordnet sein, insbesondere jeweils abwechselnd eine LED zum Aussenden von polarisiertem Licht und eine LED zum Aussenden von nicht polarisiertem Licht. Hiermit kann die erwähnte Differenzialdiagnose in einfache Weise durchgeführt werden, insbesondere ohne die Notwendigkeit eines Polfilters.

Nach einer Ausgestaltung ist der Abstandsvorsatz magnetisch an dem Bildgebungseinheitsgehäuse gehalten. Der Abstandsvorsatz kann beispielsweise aus magnetischem Edelstahl gebildet sein. Das Bildgebungseinheitsgehäuse kann ein oder mehrere Magnetelemente aufweisen zum magnetischen Halten des Abstandsvorsatzes an dem Bildgebungseinheitsgehäuse. Die Magnetelemente sind bevorzugt in einem Inneren des Bildgebungseinheitsgehäuse angeordnet, sodass kein Staub oder anderweitige Fremdkörper von außen eindringen können. Ein solches magnetisches Anbringen des Abstandsvorsatzes an dem Bildgebungseinheitsgehäuse vereinfacht den Wechsel der Abstandsvorsätze.

Nach einer Ausgestaltung weist der Abstandsvorsatz an seinem von dem Bildgebungseinheitsgehäuse abgewandten und bei einer Hautuntersuchung mit der zu untersuchenden Haut in Kontakt stehenden Ende eine Öffnung auf, wie bereits erwähnt. Die Öffnung ist gemäß einer Ausgestaltung mit einem Deckglas verschlossen. Bei der Untersuchung der Haut kann zunächst eine Immersionsflüssigkeit auf das Deckglas und/oder den zu untersuchenden Hautabschnitt aufgebracht und anschließend der Abstandsvorsatz mit dem Deckglas in Kontakt zu dem zu untersuchenden Hautabschnitt gebracht werden. Es kann somit eine Kontaktdermatoskopie durchgeführt werden. Nach einer Ausgestaltung ist die Öffnung des Abstandsvorsatzes unverschlossen, weist also insbesondere kein Deckglas auf. Ein solcher Abstandsvorsatz ist besonders geeignet für die Untersuchung von erhabenen Hautläsionen oder von Blutgefäßen, die durch Druck verschwinden würden. So kann bei der Kontaktdermatoskopie der Druck auf die untersuchten Läsionen zu einer Ausblutung (eng. exsanguination) der Gefäße führen, was die Diagnose erschweren kann. In diesem Sinne kann mit dem digitalen Dermatoskop dann eine "Nicht-Kontakt"-Dermatoskopie durchgeführt werden, wobei der die Öffnung begrenzende Öffnungsrand des Abstandsvorsatzes bei der Untersuchung allerdings weiterhin in Kontakt mit der Haut steht.

Nach einer Ausgestaltung ist das digitale Dermatoskop wasserdicht. Eine solche Wasserdichtheit kann gemäß IPX-Standard sein und kann beispielsweise über Dichtungsringe erreicht werden, insbesondere über einen zwischen der Bildgebungseinheit und dem Kopfteil angeordneten Dichtungsring und/oder einem zwischen dem Objektiv und dem Bildsensor der Bildgebungseinheit angeordneten Dichtungsring. Ein eventuell vorgesehener Lade- oder Datenübertragungsanschluss kann beispielsweise in das Griffteil eingegossen und somit wasserdicht sein.

Die erwähnten Ausgestaltungen sind miteinander kombinierbar.

Ausführungsbeispiele der Erfindung werden im Folgenden anhand von Figuren erläutert. Es zeigen schematisch:
- Figur 1: das erfindungsgemäße digitale Dermatoskop in einer perspektivischen Ansicht,
- Figur 2: das digitale Dermatoskop aus Figur 1 in einer ersten Seitenansicht,
- Figur 3: das digitale Dermatoskop aus Figur 1 in einer zweiten Seitenansicht,
- Figur 4: das digitale Dermatoskop aus Figur 1 in einer Ansicht von unten,
- Figur 5: das digitale Dermatoskop aus Figur 1 in einer Schnittansicht,
- Figur 6: die Bildgebungseinheit des digitalen Dermatoskops aus Figur 1 in einer Schnittansicht, und
- Figur 7: zwei Abstandsvorsätze für das digitale Dermatoskop aus Figur 1.

Soweit nichts anderes angegeben ist, bezeichnen im Folgenden die gleichen Bezugszeigen die gleichen Gegenstände. Begriffe wie "oben" und "Oberseite" bzw. "unten" und "Unterseite" beziehen sich auf einen Verwendungszustand des digitalen Dermatoskops, bei der die Displays der Bedienperson zugewandt und somit von dieser ablesbar sind und der Abstandsvorsatz auf der zu untersuchenden Haut aufgesetzt ist.

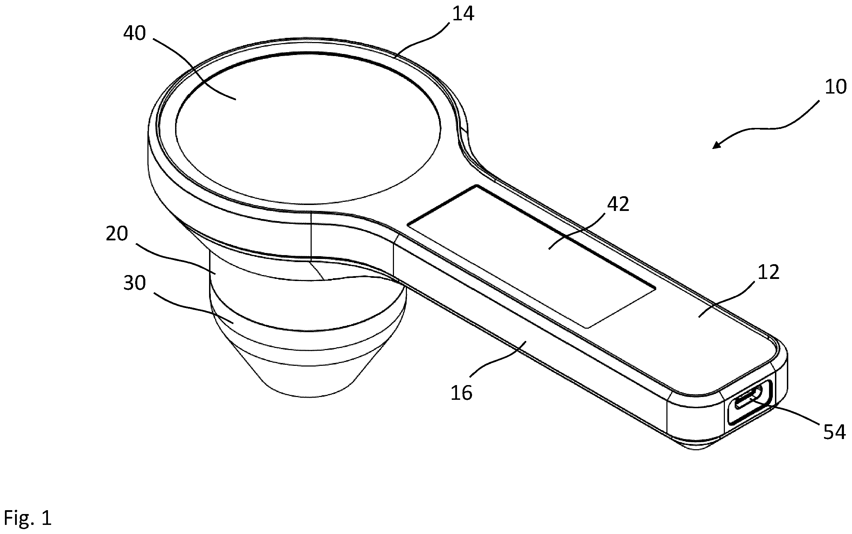

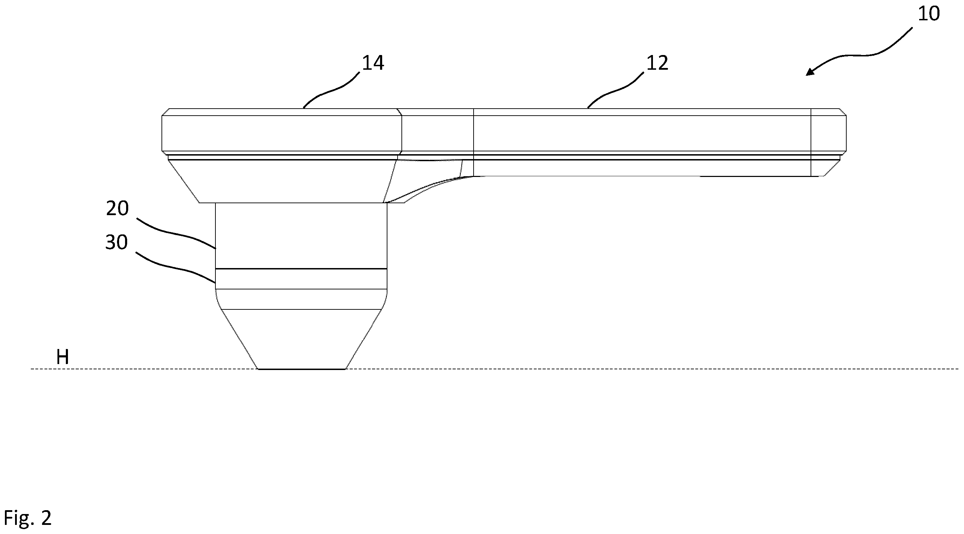

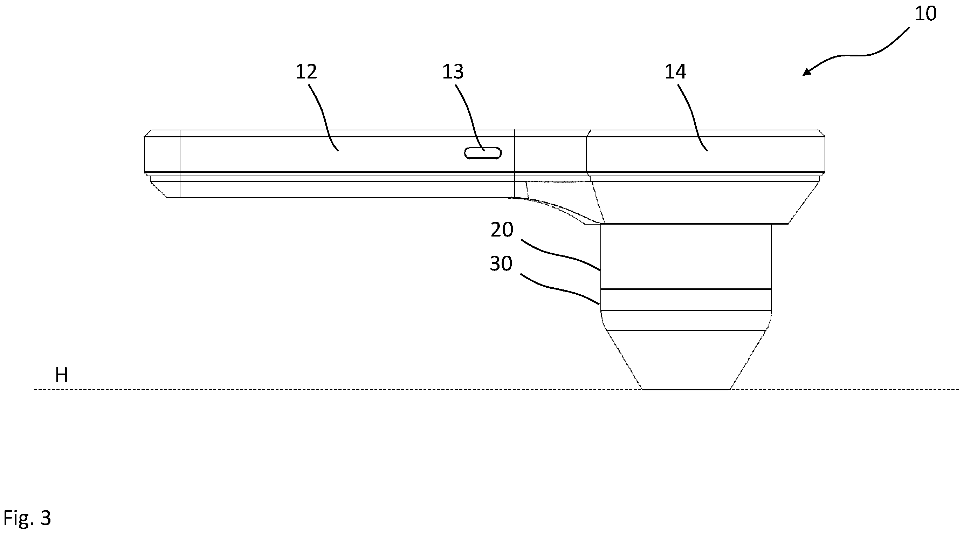

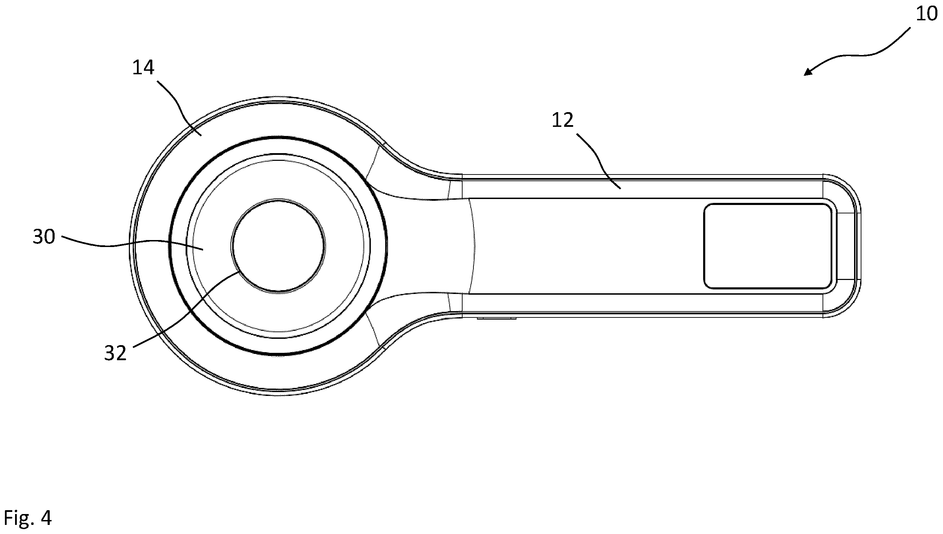

Die Figuren 1 bis 6 zeigen ein digitales Dermatoskop 10 gemäß der Erfindung. Das digitale Dermatoskop 10 umfasst ein mit der Hand einer Bedienperson umgreifbares Griffteil 12 und ein an das Griffteil 12 anschließendes Kopfteil 14. Das digitale Dermatoskop 10 ist als Handgerät ausgebildet, da eine Bedienperson es (durch Greifen des Griffteils 12) in die Hand nehmen und bestimmungsgemäß zur Untersuchung der Haut eines Patienten verwenden kann. Das Griffteil 12 und das Kopfteil 14 sind in einen Gehäuserahmen 16 eingefasst, der bevorzugt aus Aluminium gefräst ist.

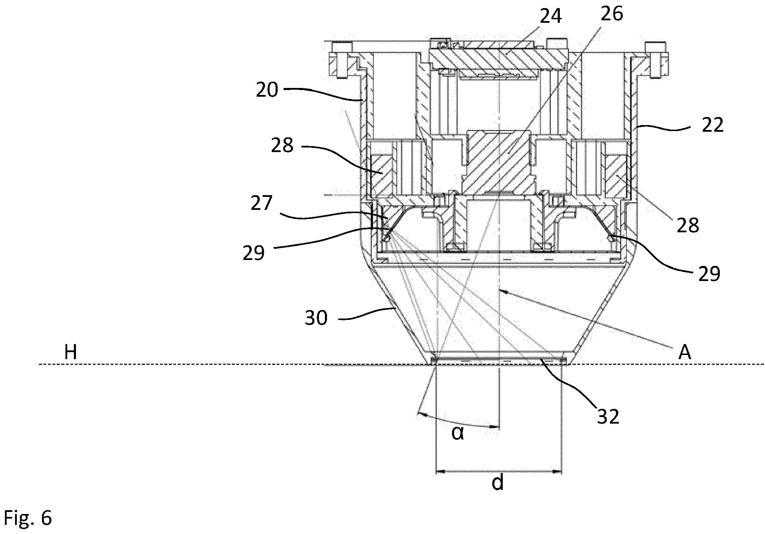

Das digitale Dermatoskop 10 umfasst weiter eine Bildgebungseinheit 20, die an einer Unterseite des Kopfteils 14 angeordnet ist, sowie einen der Bildgebungseinheit 20 vorgesetzten, an deren freiem Ende angeordneten Abstandsvorsatz 30. Die Bildgebungseinheit 20 weist ein Bildgebungseinheitsgehäuse 22, einen in dem Bildgebungseinheitsgehäuse 22 aufgenommenen Bildsensor 24, ein Objektiv 26 mit einer Festbrennweite, sowie eine einen LED-Ring umfassende Beleuchtungseinheit 27 zur Ausleuchtung auf. Die Beleuchtungseinheit 27 kann beispielsweise sechs LEDs zum Aussenden von polarisiertem Licht und sechs LEDs zum Aussenden von nicht polarisiertem Licht aufweisen, die gleichmäßig und abwechselnd über den Umfang des LED-Rings verteilt angeordnet sind. Die LEDs 29 sind in einem Winkel zu der optischen Achse A angeordnet. Der Abstandsvorsatz 30 weist eine auf die Bildgebungseinheit 20, insbesondere auf die Position des Objektivs 26, abgestimmte Länge L auf. Somit wird sichergestellt, dass die Fokusebene der Bildgebungseinheit 20 in dem Bereich einer Öffnung 32 des Abstandsvorsatzes 30 liegt. Die kreisförmige Öffnung 32 weist einen Öffnungsdurchmesser d von beispielsweise 23,8 mm auf. Aufgrund der Entfernung des Eingangs des Objektivs 26 zu der Öffnung 32 beträgt ein Öffnungswinkel α beispielsweise 20,40° (siehe Figur 6).

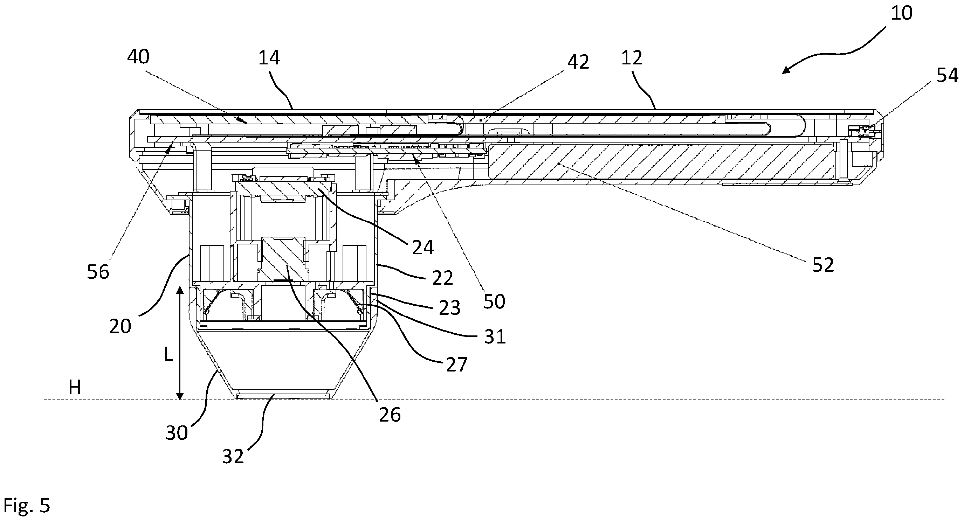

Der Abstandsvorsatz 30 ist über innerhalb des Bildgebungseinheitsgehäuses 22 angeordnete Magnetelemente 28 an dem Bildgebungseinheitsgehäuse 22 lösbar gehalten (siehe Figur 6). Wie beispielsweise in Fig. 5 ersichtlich, weist das Bildgebungseinheitsgehäuse 22 hierfür an seinem Ende eine kreisförmige Aussparung 23 auf und der Abstandsvorsatz 30 ein korrespondierend zu der kreisförmigen Aussparung 23 geformtes Ansatzstück 31, sodass, wenn der Abstandsvorsatz 30 auf das Bildgebungseinheitsgehäuse 22 aufgesetzt wird, das Ansatzstück 31 mit der kreisförmigen Aussparung 23 in Überlappung kommt. Durch Überwinden der Magnetkraft kann der Abstandsvorsatz 30 von dem Bildgebungseinheitsgehäuse 22 gelöst und durch einen anderen Abstandsvorsatz 30 ersetzt werden, beispielsweise durch einen Abstandsvorsatz mit einem anderen Öffnungsdurchmesser d der Öffnung 32.

Ein erstes Display 40 ist in dem Kopfteil 14 an dessen Oberseite angeordnet. Das erste Display 40 weist eine zu der Form des Kopfteils 14 korrespondierende kreisrunde Form auf, wie in Figur 1 ersichtlich. Zudem ist an der Oberseite des Griffteils 12 ein zweites Display 42 angeordnet, welches eine zu der Form des Griffteils 12 korrespondierende rechteckige Form aufweist. In diesem Ausführungsbeispiel erstreckt sich das zweite Display 42 über einen Großteil (mehr als 50%) der Oberseite des Griffteils 12. Beide Displays 40, 42 sind Touch-Displays.

Das digitale Dermatoskop 10 umfasst weiter interne Komponenten, darunter eine Steuereinheit 50, eine im Griffteil 12 verbaute Batterie 52, einen am Ende des Griffteils 12 verbauten Lade- und Datenübertragungsanschluss 54, sowie ein mit der Steuereinheit 50 verbundenes Trägerboard 56. Die Steuereinheit 50 umfasst insbesondere eine Leiterplatte (printed circuit board, PCB) mit einem oder mehreren Prozessoren. Die Steuereinheit 50 dient zur Ansteuerung der elektronischen Komponenten des digitalen Dermatoskops 10. Das digitale Dermatoskop 10 kann zudem auch eine Speichereinheit umfassen, insbesondere als Teil der Leiterplatte, zum Speichern von aufgenommenen Bildern. Die Batterie 12 dient zur Energieversorgung aller elektronischen Komponenten und kann über den Ladeund Datenübertragungsanschluss 54 geladen werden.

Im Verwendungszustand des digitalen Dermatoskops 10 ist das digitale Dermatoskop 10 mit seinem Abstandsvorsatz 30 auf eine zu untersuchende Hautoberfläche H aufgesetzt. Dabei kontaktiert der Abstandsvorsatz 30 die zu untersuchende Haut zumindest mit seinem die Öffnung 32 begrenzenden Öffnungsrand. Die Öffnung 32 begrenzt damit einen zu untersuchenden Hautabschnitt. Die Bedienperson kann das digitale Dermatoskop, beispielsweise über eine seitlich am Griffteil 12 angeordnete Bedientaste 13, an- und ausschalten. Im angeschalteten Zustand sendet die Beleuchtungseinheit 27 sichtbares Licht über ihre LEDs 29 aus und beleuchtet damit den zu untersuchenden Hautabschnitt, wie insbesondere in Figur 6 zu erkennen. Aufgrund der angewinkelten Stellung der LEDs 29 relativ zur optischen Achse A wird eine besonders gute Ausleuchtung des zu untersuchenden Hautabschnitts erreicht. Die beleuchtete Haut reflektiert das von der Beleuchtungseinheit 27 ausgesandte Licht und wirft dieses in das Objektiv 26, welches das aufgenommene Licht auf den Bildsensor 24 fokussiert. Basierend auf den durch den Bildsensor 24, welcher beispielsweise ein zwölf Megapixel-Sensor ist, empfangenen Signalen erstellt die Steuereinheit 50 ein digitales Bild des zu untersuchenden Hautabschnitts und zeigt dieses auf dem ersten Display 40 an. Die Steuereinheit 50 ist also dazu ausgebildet (eingerichtet, programmiert), das erste Display 40 zur Darstellung von mittels der Bildgebungseinheit 20 aufgenommenen Bildern eines zu untersuchenden Hautabschnitts anzusteuern.

Das zweite Display 42 stellt derweil Bedienelemente zum Bedienen des digitalen Dermatoskops 10 dar, beispielsweise ein Bedienelement zur Darstellung des durch die Bildgebungseinheit aufgenommenen Bildes auf dem ersten Display in anderen Farben, insbesondere in Falschfarben oder schwarz-weiß, ein Bedienelement zur Änderung des Kontrastes des auf dem ersten Display dargestellten Bildes, und/oder ein Bedienelement zum Anzeigen von zuvor aufgenommenen Bildern. Diese Bedienelemente kann die Bedienperson beispielsweise über ihren Daumen einfach erreichen, wenn sie das Griffteil 12 in der Hand hält.

Das erste Display 40 liegt in der optischen Achse A der Bildgebungseinheit 20, sodass die optische Achse A durch die Mitte des ersten Displays 40 verläuft. Dies macht die Verwendung des digitalen Dermatoskops 40 besonders intuitiv, da die Bedienperson, wie bei den ihr bereits bekannten analogen Dermatoskopen, somit den Eindruck erhält in gerader Linie "durch das Dermatoskop hindurch zu blicken". Aufgrund der kreisförmigen Ausbildung des ersten Displays 40 wird ebenfalls eine Ähnlichkeit zu einem analogen Dermatoskop hergestellt, was zu einer intuitiven Bedienung beiträgt. Zudem ermöglicht dies eine besonders effektive Hautuntersuchung, da die meisten Hauterkrankungen oder Dermatosen üblicherweise eine zumindest annähernd runde Form aufweisen.

Das erste Display 40 ist als Touch-Display dazu ausgebildet, von der Bedienperson mittels ihrer Finger auf der Displayoberfläche durchgeführte Touch-Gesten zu erkennen und in Reaktion auf das Erkennen einer vordefinierten Touch-Geste eine Aktion durchzuführen. Bevorzugt ist das Display demnach dazu ausgebildet eine Touch-Geste zu erkennen, die eine Zoom-Funktion auslöst. Somit kann die Bedienperson unmittelbar auf dem ersten Display 40 ein dort dargestelltes Bild des zu untersuchenden Hautabschnitts mittels Touch-Geste vergrößern oder verkleinern, insbesondere unter Anwendung eines Digital-Zooms. Das erste Display 40 nimmt den "Lupencharakter" eines analogen Dermatoskops auf und überträgt diesen für die Bedienperson in die digitale Welt.

Das erfindungsgemäße digitale Dermatoskop ermöglicht eine einfachere, benutzerfreundlichere Untersuchung der Haut sowie eine zuverlässigere Diagnose.

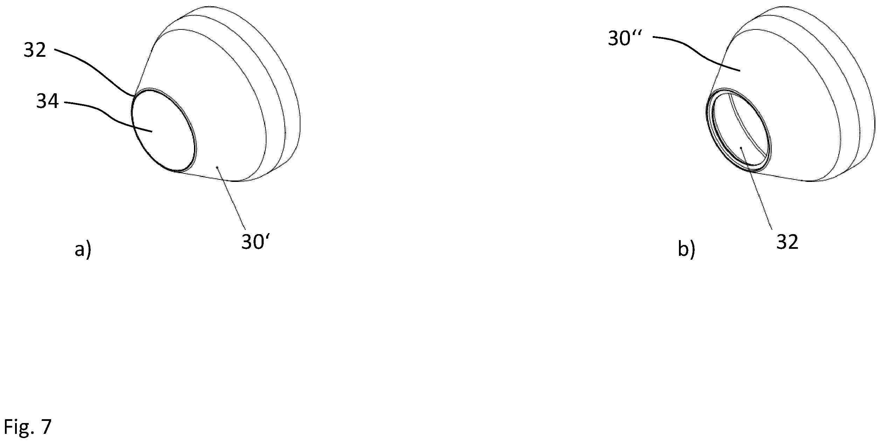

Figur 7 zeigt zwei unterschiedliche Abstandsvorsätze 30`, 30", welche für die Verwendung mit dem erfindungsgemäßen digitalen Dermatoskop 10 geeignet sind.

Bei dem in Figur 7a gezeigten Abstandsvorsatz 30` ist die Öffnung 32 mit einem Deckglas 34 verschlossen. Bei der Untersuchung der Haut wird zunächst eine Immersionsflüssigkeit auf das Deckglas 34 und/oder den zu untersuchenden Hautabschnitt aufgebracht und anschließend der Abstandsvorsatz 30' mit dem Deckglas 34 in Kontakt zu dem zu untersuchenden Hautabschnitt gebracht. Es kann somit eine Kontaktdermatoskopie durchgeführt werden.

Bei dem in Figur 7b gezeigten Abstandsvorsatz 30" ist die Öffnung 32 frei, also nicht mit einem Deckglas verschlossen. Der Abstandsvorsatz 30" ist besonders geeignet für die Untersuchung von über die umgebende Hautoberfläche erhabenen Hautläsionen oder von Blutgefäßen, die durch Druck verschwinden würden. In diesem Sinne kann mit dem digitalen Dermatoskop 10 dann eine "Nicht-Kontakt"-Dermatoskopie durchgeführt werden, wobei der die Öffnung 32 begrenzende Öffnungsrand des Abstandsvorsatzes 30" bei der Untersuchung allerdings weiterhin in Kontakt mit der Haut steht.

### Bezugszeichenliste

- 10: digitales Dermatoskop
- 12: Griffteil
- 13: Bedientaste
- 14: Kopfteil
- 16: Gehäuserahmen
- 20: Bildgebungseinheit
- 22: Bildgebungseinheitsgehäuse
- 23: Aussparung
- 24: Bildsensor
- 26: Objektiv
- 27: Beleuchtungseinheit
- 28: Magnetelemente
- 29: LEDs
- 30, 30`, 30": Abstandsvorsatz
- 31: Ansatzstück
- 32: Öffnung
- 34: Deckglas
- 40: erstes Display
- 42: zweites Display
- 50: Steuereinheit
- 52: Batterie
- 54: Lade- und Datenübertragungsanschluss
- 56: Trägerboard

- A: optische Achse
- d: Durchmesser der Öffnung 32
- H: Hautoberfläche

## Patentansprüche

1. Digitales Dermatoskop (10) zur Hautuntersuchung, wobei das digitale Dermatoskop (10) als Handgerät ausgebildet ist, das digitale Dermatoskop (10) umfassend ein mit der Hand umgreifbares Griffteil (12) und ein an das Griffteil (12) anschließendes, eine Bildgebungseinheit (20) aufweisendes Kopfteil (14), wobei die Bildgebungseinheit (20) ein an einer Unterseite des Kopfteils (14) angeordnetes Bildgebungseinheitsgehäuse (22), einen in dem Bildgebungseinheitsgehäuse (22) aufgenommenen Bildsensor (24), sowie ein Objektiv (26) aufweist, wobei endseitig an dem Bildgebungseinheitsgehäuse (22) ein auswechselbarer Abstandsvorsatz (30) angeordnet ist, der bei einer Hautuntersuchung die zu untersuchende Haut kontaktiert, das digitale Dermatoskop (10) weiter umfassend eine Steuereinheit (50) sowie ein an einer Oberseite des Kopfteils (14) angeordnetes erstes Display (40), wobei die Steuereinheit (50) dazu ausgebildet ist, das erste Display (40) zur Darstellung von mittels der Bildgebungseinheit (20) aufgenommenem Bildern eines zu untersuchenden Hautabschnitts anzusteuern.

2. Digitales Dermatoskop (10) nach Anspruch 1, wobei das erste Display (40) eine zu der Form des Kopfteils (14) korrespondierende Form aufweist, insbesondere kreisrunde Form.

3. Digitales Dermatoskop (10) nach Anspruch 1 oder 2, wobei die Steuereinheit (50) dazu ausgebildet ist, eine Live-Ansicht des zu untersuchenden Hautabschnitts auf dem ersten Display (40) darzustellen.

4. Digitales Dermatoskop (10) nach einem der vorhergehenden Ansprüche, wobei die Steuereinheit (50) dazu ausgebildet ist, ein auf dem ersten Display (40) dargestelltes Bild des zu untersuchenden Hautabschnitts in Reaktion auf eine Bedieneingabe mittels eines digitalen Zooms zu vergrößern.

5. Digitales Dermatoskop (10) nach einem der vorhergehenden Ansprüche, weiter umfassend ein an einer Oberseite des Griffteils (12) angeordnetes zweites Display (42), welches als Touch-Display ausgebildet ist, zur Darstellung von Bedienelementen zum Bedienen des digitalen Dermatoskops (10).

6. Digitales Dermatoskop (10) nach einem der vorhergehenden Ansprüche, wobei das digitale Dermatoskop (10) einen das Griffteil (12) und das Kopfteil (14) einfassenden Gehäuserahmen (16) aufweist, wobei der Gehäuserahmen (16) bevorzugt aus Aluminium besteht, insbesondere aus gefrästem Aluminium.

7. Digitales Dermatoskop (10) nach einem der vorhergehenden Ansprüche, wobei das Bildgebungseinheitsgehäuse (22) aus Aluminium besteht, insbesondere aus gefrästem Aluminium.

8. Digitales Dermatoskop (10) nach einem der vorhergehenden Ansprüche, wobei die Bildgebungseinheit (20) einen Polarisationsfilter umfasst.

9. Digitales Dermatoskop (10) nach einem der vorhergehenden Ansprüche, umfassend eine in dem Griffteil (12) angeordnete wiederaufladbare Batterie (52), insbesondere eine Lithium-Polymer-Batterie, zur Energieversorgung der elektronischen Bauteile des digitalen Dermatoskops (10), insbesondere der Steuereinheit (50) und des ersten Displays (40).

10. Digitales Dermatoskop (10) nach einem der vorhergehenden Ansprüche, umfassend eine nicht-flüchtige Speichereinheit zur elektronischen Speicherung von durch die Bildgebungseinheit (20) aufgenommenen Bildern und/oder Videos.

11. Digitales Dermatoskop (10) nach einem der vorhergehenden Ansprüche, umfassend einen Anschluss (54), bevorzugt angeordnet an einem Ende des Griffteils (12), zum Aufladen einer Batterie des digitalen Dermatoskops (10) und/oder zum Herstellen einer Datenverbindung zwischen dem digitalen Dermatoskop (10) und einem elektronischen Gerät, insbesondere einem Tablet.

12. Digitales Dermatoskop (10) nach einem der vorhergehenden Ansprüche, umfassend eine Kommunikationseinheit zur kabellosen Kommunikation mit einem elektronischen Gerät, insbesondere einem Tablet.

13. Digitales Dermatoskop (10) nach einem der vorhergehenden Ansprüche, wobei die Bildgebungseinheit (20) eine Beleuchtungseinheit (27) mit mehreren ringförmig angeordneten Beleuchtungselementen (29), insbesondere LEDs, aufweist zur Beleuchtung des zu untersuchenden Hautabschnitts.

14. Digitales Dermatoskop (10) nach Anspruch 13, wobei die Beleuchtungselemente (29) in einem Winkel zur optischen Achse (A) angeordnet sind.

15. Digitales Dermatoskop (10) nach einem der vorhergehenden Ansprüche, wobei der Abstandsvorsatz (30) magnetisch an dem Bildgebungseinheitsgehäuse (22) gehalten ist.

## Geänderte Patentansprüche

### Geänderte Patentansprüche gemäss Regel 137(2) EPÜ.

1. Digitales Dermatoskop (10) zur Hautuntersuchung, wobei das digitale Dermatoskop (10) als Handgerät ausgebildet ist, das digitale Dermatoskop (10) umfassend ein mit der Hand umgreifbares Griffteil (12) und ein an das Griffteil (12) anschließendes, eine Bildgebungseinheit (20) aufweisendes Kopfteil (14), wobei die Bildgebungseinheit (20) ein an einer Unterseite des Kopfteils (14) angeordnetes Bildgebungseinheitsgehäuse (22), einen in dem Bildgebungseinheitsgehäuse (22) aufgenommenen Bildsensor (24), sowie ein Objektiv (26) aufweist, wobei endseitig an dem Bildgebungseinheitsgehäuse (22) ein auswechselbarer Abstandsvorsatz (30) angeordnet ist, der bei einer Hautuntersuchung die zu untersuchende Haut kontaktiert, das digitale Dermatoskop (10) weiter umfassend eine Steuereinheit (50) sowie ein an einer Oberseite des Kopfteils (14) angeordnetes erstes Display (40), wobei die Steuereinheit (50) dazu ausgebildet ist, das erste Display (40) zur Darstellung von mittels der Bildgebungseinheit (20) aufgenommenem Bildern eines zu untersuchenden Hautabschnitts anzusteuern, wobei das erste Display (40) eine zu der Form des Kopfteils (14) korrespondierende kreisrunde Form aufweist.

2. Digitales Dermatoskop (10) nach Anspruch 1, wobei die Steuereinheit (50) dazu ausgebildet ist, eine Live-Ansicht des zu untersuchenden Hautabschnitts auf dem ersten Display (40) darzustellen.

3. Digitales Dermatoskop (10) nach einem der vorhergehenden Ansprüche, wobei die Steuereinheit (50) dazu ausgebildet ist, ein auf dem ersten Display (40) dargestelltes Bild des zu untersuchenden Hautabschnitts in Reaktion auf eine Bedieneingabe mittels eines digitalen Zooms zu vergrößern.

4. Digitales Dermatoskop (10) nach einem der vorhergehenden Ansprüche, weiter umfassend ein an einer Oberseite des Griffteils (12) angeordnetes zweites Display (42), welches als Touch-Display ausgebildet ist, zur Darstellung von Bedienelementen zum Bedienen des digitalen Dermatoskops (10).

5. Digitales Dermatoskop (10) nach einem der vorhergehenden Ansprüche, wobei das digitale Dermatoskop (10) einen das Griffteil (12) und das Kopfteil (14) einfassenden Gehäuserahmen (16) aufweist, wobei der Gehäuserahmen (16) bevorzugt aus Aluminium besteht, insbesondere aus gefrästem Aluminium.

6. Digitales Dermatoskop (10) nach einem der vorhergehenden Ansprüche, wobei das Bildgebungseinheitsgehäuse (22) aus Aluminium besteht, insbesondere aus gefrästem Aluminium.

7. Digitales Dermatoskop (10) nach einem der vorhergehenden Ansprüche, wobei die Bildgebungseinheit (20) einen Polarisationsfilter umfasst.

8. Digitales Dermatoskop (10) nach einem der vorhergehenden Ansprüche, umfassend eine in dem Griffteil (12) angeordnete wiederaufladbare Batterie (52), insbesondere eine Lithium-Polymer-Batterie, zur Energieversorgung der elektronischen Bauteile des digitalen Dermatoskops (10), insbesondere der Steuereinheit (50) und des ersten Displays (40).

9. Digitales Dermatoskop (10) nach einem der vorhergehenden Ansprüche, umfassend eine nicht-flüchtige Speichereinheit zur elektronischen Speicherung von durch die Bildgebungseinheit (20) aufgenommenen Bildern und/oder Videos.

10. Digitales Dermatoskop (10) nach einem der vorhergehenden Ansprüche, umfassend einen Anschluss (54), bevorzugt angeordnet an einem Ende des Griffteils (12), zum Aufladen einer Batterie des digitalen Dermatoskops (10) und/oder zum Herstellen einer Datenverbindung zwischen dem digitalen Dermatoskop (10) und einem elektronischen Gerät, insbesondere einem Tablet.

11. Digitales Dermatoskop (10) nach einem der vorhergehenden Ansprüche, umfassend eine Kommunikationseinheit zur kabellosen Kommunikation mit einem elektronischen Gerät, insbesondere einem Tablet.

12. Digitales Dermatoskop (10) nach einem der vorhergehenden Ansprüche, wobei die Bildgebungseinheit (20) eine Beleuchtungseinheit (27) mit mehreren ringförmig angeordneten Beleuchtungselementen (29), insbesondere LEDs, aufweist zur Beleuchtung des zu untersuchenden Hautabschnitts.

13. Digitales Dermatoskop (10) nach Anspruch 12, wobei die Beleuchtungselemente (29) in einem Winkel zur optischen Achse (A) angeordnet sind.

14. Digitales Dermatoskop (10) nach einem der vorhergehenden Ansprüche, wobei der Abstandsvorsatz (30) magnetisch an dem Bildgebungseinheitsgehäuse (22) gehalten ist.
